# EUROPÄISCHE PATENTANMELDUNG

(11) **EP 2 324 817 A2**
(43) Veröffentlichungstag der Anmeldung: **25.05.2011**
(21) Anmeldenummer: 10160413.0
(22) Anmeldetag: 20.04.2010
(51) Int. Cl.: A61K 8/37, A61K 8/92, A61Q 5/10, A61Q 5/12

(54) **Pflegende Haarfärbemittel**

(30) Priorität: 21.07.2009 DE 102009027895
(71) Anmelder: Henkel AG & Co. KGaA, 40589 Düsseldorf (DE)
(72) Erfinder: Reichert, Anja, 40629, Düsseldorf (DE); Ehlert, Manuela, 51379, Leverkusen (DE)

(57) **Zusammenfassung**

Gegenstand der Erfindung ist ein Mittel zur oxidativen Farbveränderung von keratinischen Fasern, insbesondere menschlichen Haaren, welches in einem kosmetischen Träger mindestens ein Oxidationsfarbstoffvorprodukt enthält, wobei das Mittel zusätzlich mindestens einen Fettsäureester einer ungesättigten Fettsäure mit einem Fettalkohol und mindestens ein Fettsäuretriglycerid enthält.

## Beschreibung

Gegenstand der Erfindung ist ein Mittel zur oxidativen Farbveränderung von keratinischen Fasern, welches in einem kosmetischen Träger neben mindestens einem Oxidationsfarbstoffvorprodukt eine Kombination pflegender Fettkörper enthält. Die erfindungsgemäßen Mittel eignen sich insbesondere zur Reduktion der Haarschädigung und zur Verbesserung der Haaroberfläche bei der oxidativen Färbung. Darüber hinaus betrifft die Erfindung die Verwendung des Mittels zur Verringerung der Haarschädigung und zur Verbesserung der Haaroberfläche.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Diese Mittel sollen neben der gewünschten Färbe- und Formleistung möglichst minimale Schädigungen auf dem Haar hervorrufen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln oder von Luftsauerstoff untereinander die eigentlichen Farbstoffe ausbilden. Die Oxidationsfärbemittel zeichnen sich durch hervorragende, lang anhaltende Färbeergebnisse aus. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen. Schließlich hat ein weiteres Färbeverfahren große Beachtung gefunden, bei dem Vorstufen des natürlichen Haarfarbstoffes Melanin auf das Substrat, z. B. Haare, aufgebracht werden, wobei diese dann im Rahmen oxidativer Prozesse im Haar naturanaloge Farbstoffe ausbilden.

Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Oxidative Färbemittel enthalten in der Regel Wasserstoffperoxid als Oxidationsmittel. Dies führt neben dem gewünschten kosmetischen Effekt zu oxidativen Schädigungen der Haaroberfläche. Auch führt der üblicherweise in solchen Mitteln basisch eingestellte pH-Wert dazu, dass die Struktur des Haares oxidativ geschädigt wird. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids ist, desto stärkere Schädigungen werden in der Regel auf der Keratinfaser hervorgerufen. Insbesondere Spliss oder gar Bruch der Faser sind vom Verbraucher höchst unerwünschte Begleiterscheinungen einer Blondierung, die insbesondere bei mehrfacher Wiederholung des oxidativen Färbeprozesses auftreten können. Es ist daher besonders wünschenswert, Färbemittel bereitzustellen, die neben einer guten Färbeleistung einen positiven Beitrag zur Verbesserung der Faserstruktur leisten und damit gleichzeitig als Pflege- oder Konditioniermittel wirken. Somit könnte auf die sonst häufig erforderliche, zusätzliche pflegende Nachbehandlung verzichtet werden, die sowohl hinsichtlich Anwendungskomfort als zusätzlichen Behandlungsschritt als auch hinsichtlich Verpackungsökonomie mit Nachteilen behaftet ist.

In der Vergangenheit erwiesen sich Versuche, handelsübliche Pflegestoffe in Färbemittel einzuarbeiten, häufig als unzureichend und wenig erfolgreich, da übliche Pflegestoffe für keratinische Fasern insbesondere unter den harschen oxidativen Färbebedingungen mangelnde Stabilität besitzen und nur eingeschränkt Wirkung zeigen. Es besteht somit weiterhin ein großer Verbesserungsbedarf für solche weniger schädigenden oder pflegenden Färbemittel.

Aufgabe der vorliegenden Erfindung ist daher die Bereitstellung kosmetischer Mittel zur Farbveränderung keratinischer Fasern, die eine verringerte Haarschädigung aufweisen. Eine solche verringerte Haarschädigung lässt sich an der Haaroberfläche beispielsweise durch eine Verringerung von Haarbruch oder Spliss nachweisen. Weiterhin ist auch eine verringerte Kämmarbeit ein Nachweis für verbesserte Haaroberfläche. Unter Kämmbarkeit versteht sich erfindungsgemäß sowohl die Kämmbarkeit der nassen Faser, als auch die Kämmbarkeit der trockenen Faser. Als Maß für die Kämmbarkeit dient die aufgewendete Kämmarbeit oder die aufgewendete Kraft während des Kämmvorganges eines Faserkollektivs. Die Meßparameter können durch den Fachmann sensorisch beurteilt oder durch Messeinrichtungen quantifiziert werden.

In nicht vorhersehbarer Weise konnte nun gefunden werden, dass farbverändernde Mittel, die neben einem Oxidationsfarbstoffvorprodukt als farbverändernden Komponente mindestens eine spezielle spezielle Kombination aus mindestens zwei unterschiedlichen, pflegenden Fettkörpern, umfassend einen ungesättigten Fettsäureester und ein Fettsäuretriglycerid, enthalten, die oben genannten Nachteile vermeiden. Bedingt durch die pflegende Wirkung bei Anwendung des erfindungsgemäßen Mittels kann die Haarschädigung hierdurch deutlich verbessert werden.

Ein erster Gegenstand der Erfindung ist daher ein Mittel zur oxidativen Farbveränderung von keratinischen Fasern, insbesondere menschlichen Haaren, welches in einem kosmetischen Träger mindestens ein Oxidationsfarbstoffvorprodukt enthält und dadurch gekennzeichnet ist, dass das Mittel zusätzlich
(a) mindestens einen Fettsäureester einer ungesättigten Fettsäure mit einem Fettalkohol und
(b) mindestens ein Fettsäuretriglycerid enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten wesentlichen Inhaltsstoff enthält das erfindungsgemäße Mittel mindestens einen Fettsäureester einer ungesättigten Fettsäure mit einem Fettalkohol.

Unter einer Fettsäure sind erfindungsgemäß ungesättigte, gegebenenfalls verzweigte und/oder substituierte d₁₀-C₂₆-Fettsäuren zu verstehen. Bevorzugt handelt es sich bei der ungesättigten Fettsäure um eine Fettsäure, deren C-C-Doppelbindung und/oder C-C-Doppelbindungen die Cis-Konfiguration aufweisen. Erfindungsgemäß geeignete Fettsäuren sind Decensäure, Dodecensäure, Tetradecensäure, Palmitoleinsäure, Ölsäure, Petroselinsäure, Elaidinsäure, Linolsäure, Linolensäure, Arachidonsäure, Gadoleinsäure (Eicosensäure), Erucasäure (Docosensäure) und Nervonsäure (Tetracosensäure) sowie deren Mischungen.

Eine weitere Ausführungsform dieses Erfinudngsgegenstands ist **dadurch gekennzeichnet, dass** sich der ungesättigte Fettsäurenanteil von Ester (a) von ungesättigten C₂₀ bis C₂₄-Fettsäuren ableitet. Besonders bevorzugt sind hierbei wiederum sogenannte ω-9-Fettsäuren, welche die Doppelbindung am C9-Atom, gerechnet vom CH₃-Terminus der Fettsäure, tragen und sich dadurch durch besondere Oxidationsstabilität auszeichnen. Weiterhin kann als Fettsäure ein Gemisch von Fettsäuren unterschiedlicher Kohlenstoffkette eingesetzt werden, insbesondere wenn es sich um Fettsäuren natürlichen Ursprungs handelt. Besonders bevorzugte Fettsäureanteile der Ester (a) sind Gadoleinsäure und/oder Erucasäure.

Bei einem Fettalkohol im Sinne der Erfindung handelt es sich um einen C₆-C₂₂-Monohydroxyalkohol, welcher einen gesättigten oder ungesättigten Kohlenstoffrest mit einem Grundkörper aus 6 bis 22 Kohlenstoffatomen aufweist, und welcher gegebenenfalls verzweigt sein kann.

Eine weitere Ausführungsform der vorliegenden Erfindung ist **dadurch gekennzeichnet, dass** als Ester (a) ein Ester einer ungesättigten Fettsäure mit einem ungesättigten Fettalkohol eingesetzt wird. Bevorzugt wird der ungesättigte Fettalkohol ausgewählt aus der Gruppe, die gebildet wird aus Palmoleylalkohol, Oleylalkohol, Elaidylalkohol, Petroselinylalkohol, Linoleylalkohol, Linolenylalkohol, Elaeostearylalkohol, Arachidonylalkohol, Gadoleylalkohol (Eicosenylalkohol) und Erucylalkohol (Docosenylalkohol). Weiterhin kann als Fettalkohol ein Gemisch von Fettalkoholen unterschiedlicher Kohlenstoffkette eingesetzt werden, insbesondere wenn es sich um Fettalkohole natürlichen Ursprungs handelt.

Solche Mittel, die dadurch gekennzeichnet sind, dass sich der Fettalkoholanteil von Ester (a) von ungesättigten, linearen C₁₈ bis C₂₄-Fettalkoholen, insbesondere von Gadoleylalkohol und/oder Erucylalkohol, ableitet, sind erfindungsgemäß besonders bevorzugt.

Insbesondere ungesättigte Fettsäureester (a), die eine Gesamtkohlenstoffanzahl von 40 bis 42 Kohlenstoffen ausweisen, sind erfindungsgemäß bevorzugt.

Das aus den Samen von *Simmondsia Chinensis* durch Pressung gewonnene Öl (Jojobaöl) besteht überwiegend aus Docosenyleicosenoat, Eicosenyleicosenoat und Eicosenyldocosenoat und ist daher reich an solchen bevorzugten, ungesättigten Fettsäureestern (a).

Eine weitere Ausführungsform der vorliegenden Erfindung ist daher **dadurch gekennzeichnet, dass** das Mittel als Fettsäureester (a) das Öl von Samen von *Simmondsia chinensis* enthält.

Als weiteren, wesentlichen Inhaltsstoff enthält das erfindungsgemäße Mittel mindestens ein Fettsäuretriglycerid. Darunter ist erfindungsgemäß ein Ester des Triols Glycerin mit drei Äquivalenten von Fettsäure zu verstehen. Dabei können sowohl strukturgleiche wie unterschiedliche Fettsäuren in einem Molekül Triglycerid an der Esterbildung beteiligt sein.

In einer Ausführungsform der vorliegenden Erfindung enthält das Mittel als Fettsäuretriglycerid (b) ein gemischtes Glycerid von gesättigten und ungesättigten, C₁₆ bis C₂₀-Fettsäuren.

Bevorzugte gesättigten und ungesättigten, C₁₆ bis C₂₀-Fettsäuren sind dabei Palmitinsäure, Palmitoleinsäure, Stearinsäure, Ölsäure, Petroselinsäure, Elaeosterinsäure, Elaidinsäure, Linolsäure, Linolensäure, Arachinsäure, Arachidonsäure, Gadoleinsäure (Eicosensäure) sowie deren Mischungen.

Es hat sich herausgestellt, dass insbesondere Triglyceride mit einem erhöhten Anteil an ungesättigten C₁₆ bis C₂₀-Fettsäuren als Fettsäurekomponente erfindungsgemäß vorteilhaft eingesetzt werden können. Vorzugsweise enthalten die Fettsäuretriglyceride (b) mindestens einen Anteil von 40 mol-% an ungesättigten C₁₆ bis C₂₀-Fettsäuren.

Das Kernöl bzw. die Kernbutter, welches nachfolgend allgemein als Öl bezeichnet wird und aus den Saaten von *Mangifera Indica* (Mango) durch Pressung gewonnen werden kann, zeichnet sich durch einen solchen erhöhten Anteil an ungesättigten C₁₆ bis C₂₀-Fettsäuren als Fettsäurekomponente aus.

Eine weitere Ausführungsform der vorliegenden Erfindung ist daher **dadurch gekennzeichnet, dass** das Mittel als Fettsäuretriglycerid (b) das Öl aus den Samen von *Mangifera Indica* enthält.

Erfindungsgemäß bevorzugte Mittel sind **dadurch gekennzeichnet, dass** das Mittel das/die Fettsäuretriglycerid(e) (b) in einem Gewichtsanteil von 0,01 bis 15,0 Gew.-%, bevorzugt von 0,05 bis 10,0 Gew.-% und insbesondere von 0,1 bis 5,0 Gew.-%, jeweils bezogen auf das Gesamtgewicht des anwendungsbereiten Mittels, enthält.

In einer weiteren Ausführungsform des ersten Erfindungsgegenstands enthält das Mittel zur oxidativen Farbveränderung von keratinischen Fasern, insbesondere menschlichen Haaren, daher in einem kosmetischen Träger mindestens ein Oxidationsfarbstoffvorprodukt und zusätzlich als Fettsäureester (a) das Öl von Samen von *Simmondsia chinensis* und als Fettsäuretriglycerid das Öl aus Samen von *Mangifera Indica.*

Die erfindungsgemäßen Mittel enthalten weiterhin als farbverändernde Komponenten mindestens ein Oxidationsfarbstoffvorprodukt.

Die Oxidationsfarbstoffvorprodukte werden bevorzugt in einer Menge von 0,005 bis 25 Gew.-%, bevorzugt von 0,05 bis 20 Gew.-% und besonders bevorzugt von 0,1 bis 15 Gew.-%, jeweils bezogen auf das anwendungsbereite Oxidationsfärbemittel, verwendet.

In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate, einzusetzen.

Erfindungsgemäße Entwicklerkomponenten werden ausgewählt aus p-Phenylendiamin, zweikernigen Entwicklerkomponenten, p-Aminophenol, o-Aminophenol, heterocyclischen Entwicklerkomponenten und/oder den Derivaten vorstehender Substanzklassen. Die Entwicklerkomponenten werden bevorzugt in einer Menge von 0,005 bis 20 Gew.-%, vorzugsweise 0,1 bis 15 Gew.-%, jeweils bezogen auf das anwendungsbereite Oxidationsfärbemittel, verwendet.

Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)tetramethylendiamin, N,N'-Bis-[4-(methylamino)phenyl]-tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder einem der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-[(2-hydroxyethyl)aminomethyl]phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol oder einem der physiologisch verträglichen Salze dieser Verbindungen.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidinderivate sind die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazolderivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze.

Bevorzugte Pyrazolopyrimidinderivate sind insbesondere die Derivate des Pyrazolo[1,5-a]pyrimidin und dessen tautomeren Formen, sofern ein tautomeres Gleichgewicht besteht. Die Pyrazolo[1,5-a]pyrimidinen sind insbesondere ausgewählt aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]-ethanol, 2-[(7-Aminopyrazolo[1,5-a]pyrimidin-3-yl)-(2-hydroxyethyl)amino]ethanol; 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihren physiologisch verträglichen Salzen und ihren tautomeren Formen, wenn ein tautomeres Gleichgewicht vorhanden ist.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Erfindungsgemäße Kupplerkomponenten werden bevorzugt ausgewählt aus m-Aminophenol, m-Diaminobenzol, o-Diaminobenzol, o-Aminophenol, Naphthalinderivaten mit mindestens einer Hydroxygruppe, Di- beziehungsweise Trihydroxybenzol, Pyridin, Pyrimidin, Monohydroxy- bzw. Monoaminoindol, Monohydroxy- bzw. Monoaminoindolin, Pyrazolon, Benzomorpholin, Chinoxalin und/oder den Derivaten der vorstehenden Substanzklassen. Die Kupplerkomponenten werden bevorzugt in einer Menge von 0,005 bis 20 Gew.-%, vorzugsweise 0,1 bis 15 Gew.-%, jeweils bezogen auf das anwendungsbereite Oxidationsfärbemittel, verwendet.

Die erfindungsgemäß bevorzugten m-Aminophenole bzw. deren Derivate werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-methylaminobenzol, 3-Ethylamino-4-methylphenol, 2,4-Dichlor-3-aminophenol und den physiologisch verträglichen Salzen der vorstehend genannten Verbindungen.

Die erfindungsgemäß bevorzugten m-Diaminobenzole bzw. deren Derivate werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und den physiologisch verträglichen Salzen der genannten Verbindungen.

Die erfindungsgemäß bevorzugten o-Diaminobenzole bzw. deren Derivate werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen der genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß bevorzugten Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß bevorzugten Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß bevorzugten Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Weiterhin können die erfindungsgemäßen Mittel als zusätzliche farbverändernde Komponente, insbesondere zur Nuancierung, mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe sind als anionische, kationische und nichtionische direktziehende Farbstoffe bekannt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonefarbstoffe wie HC Blue 16 (Bluequat B), sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

In einer weiteren Ausführungsform der vorliegenden Erfindung wird als zusätzliche farbverändernde Komponente mindestens eine Farbstoffvorstufe naturanaloger Farbstoffe zugegeben.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%.

Bevorzugte Derivate des Indolins sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure und besonders bevorzugt 5,6-Dihydroxyindolin. Bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure sowie insbesondere 5,6-Dihydroxyindol.

Eine weitere Möglichkeit zur Farbgebung bietet die Verwendung von Färbemitteln, welche sogenannte Oxofarbstoffvorprodukte enthalten. Als zusätzliche farbverändernde Komponente können daher in einer weiteren Ausführungsform auch Oxofarbstoffvorprodukte eingesetzt werden. Oxofarbstoffvorprodukte werden bevorzugt als Kombination aus mindestens einer Verbindung (Oxo1), die mindestens eine reaktive Carbonylgruppe enthält, mit mindestens einer Verbindung (Oxo2), ausgewählt aus C,H-aciden Verbindungen und/oder aus Verbindungen mit primärer oder sekundärer Aminogruppe oder Hydroxygruppe, eingesetzt.

Die vorgenannten Komponenten (Oxo1) und (Oxo2) sind im Allgemeinen selbst keine Farbstoffe, und eignen sich daher jede für sich genommen allein nicht zur Färbung keratinhaltiger Fasern. In Kombination bilden sie in dem nichtoxidativen Prozess der sogenannten Oxofärbung die eigentlichen Farbstoffe aus. Die resultierenden Färbungen besitzen teilweise Farbechtheiten auf der keratinhaltigen Faser, die mit denen der Oxidationsfärbung vergleichbar sind.

Es ist jedoch erfindungsgemäß bevorzugt, wenn (Oxo2) nur unter C,H-aciden Verbindungen ausgewählt werden. Die voranstehend genannten Verbindungen (Oxo1) sowie (Oxo2) werden, wenn sie zum Einsatz kommen, jeweils vorzugsweise in einer Menge von 0,001 bis 10 Gew.-%, insbesondere von 0,01 bis 5 Gew.-%, jeweils bezogen auf Gesamtgewicht des anwendungsbereiten Mittels, verwendet.

Es ist nicht erforderlich, dass Oxidationsfarbstoffvorprodukte, direktziehende Farbstoffe, Oxofarbstoffvorprodukte oder naturanaloge Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die Untersuchungen der Anmelderin im Zuge dieser Erfindung zeigten, dass sich die Haarschädigung während der oxidativen Haarfärbung noch weiter reduzieren lässt, wenn das erfindungsgemäße Mittel zusätzlich mindestens einen verzweigten Fettalkohol enthält.

Eine weitere Ausführungsform des ersten Erfindungsgegenstands ist daher **dadurch gekennzeichnet, dass** das Mittel zusätzlich einen verzweigten C₈-C₂₄-Fettalkohol enthält.

Geeignete verzweigte C₈-C₂₄-Fettalkohole sind insbesondere sogenannte Guerbet-Alkohole, welche durch Kondensation zweier kurzkettiger Alkohole herstellbar sind. Hierzu gehören insbesondere 2-Ethyl-hexan-1-ol, 2-Propyl-heptan-1-ol, 2-Butyl-octan-1-ol, 2-Pentyl-nonan-1-ol, 2-Hexyl-decan-1-ol, 2-Heptyl-undecan-1-ol, 2-Octyl-dodecan-1-ol sowie 2-Decyl-tetradecan-1-ol. Besonders bevorzugt sind hierbei 2-Hexyl-decan-1-ol und 2-Octyl-dodecan-1-ol, welche unter den Handelsnamen Eutanol G16 (2-Hexyl-decan-1-ol) bzw. Eutanol G oder Eutanol G20 (2-Octyldodecan-1-ol) vertrieben werden. Ganz besonders bevorzugt enthält das Mittel 2-Octyl-dodecan-1-ol.

In einer besonderen Ausführungsform enthält das Mittel den verzweigten Fettalkohol in einem Gewichtsanteil von 0,01 bis 15,0 Gew.-%, bevorzugt von 0,05 bis 10,0 Gew.-% und insbesondere von 0,1 bis 5,0 Gew.-%, jeweils bezogen auf das Gesamtgewicht des anwendungsbereiten Mittels. Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist.

Eine weitere Ausführungsform der Erfindung ist daher ein Mittel, welches zusätzlich ein Oxidationsmittel, ausgewählt aus Wasserstoffperoxid sowie seinen festen Anlagerungsprodukten an organische und anorganische Verbindungen, enthält. Als feste Anlagerungsprodukte kommen erfindungsgemäß insbesondere die Anlagerungsprodukte an Harnstoff, Melamin, Polyvinylpyrrolidinon sowie Natriumborat in Frage.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 0,8 bis 6 Gew.-%, jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen **dadurch gekennzeichnet, dass** die fließfähige Oxidationsmittelzubereitung, bezogen auf ihr Gewicht, 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Farbveränderungsmittel als Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, lodide, Chinone oder Metallionen.

Soll helles oder aufgehelltes Haar bei der Behandlung eine Auffrischung des Aufhelleffekts erfahren, so kann es vorteilhaft sein, Wasserstoffperoxid und /oder eines seiner festen Anlagerungsprodukte in ein Shampoo oder ein ähnliches Behandlungsmittel einzuarbeiten. Damit wird jedoch in der Regel nur eine geringe Aufhellung erzielt. Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und anorganischen Persulfaten eingesetzt, woraus eine Steigerung des Aufhellvermögens der Mittel resultiert. Bevorzugte Persulfatsalz sind Ammoniumperoxodisulfat, Kaliumperoxodisulfat und Natriumperoxodisulfat.

Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Der Einsatz von Persulfatsalzen bzw. Peroxodisulfatsalzen erfolgt in der Regel in Form eines gegebenenfalls entstaubten Pulvers oder eines in Form gepressten Formkörpers.
Bei einer Anwendung von zusätzlichen Oxidationsmitteln wird das eigentliche Färbemittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt, sowie einer Zubereitung, enthaltend das zusätzliche Oxidationsmittel, insbesondere Wasserstoffperoxid, hergestellt. Das erfindungsgemäße Mittel kann dabei die Kombination aus mindestens einem Fettsäureester einer ungesättigten Fettsäure mit einem Fettalkohol und mindestens einem Fettsäuretriglycerid in der Zubereitung mit Oxidationsfarbstoffvorprodukten und/oder in der Oxidationsmittelzubereitung enthalten. Bevorzugt enthält die Zubereitung mit Oxidationsfarbstoffvorprodukten die Kombination aus Fettsäureester und Fettsäuretriglycerid.

Wird eine starke Aufhellung gewünscht, so ist es erfindungsgemäß bevorzugt, wenn zusätzlich eine Blondierzubereitung, enthaltend mindestens ein anorganisches, der Oxidationsmittelzubereitung vor Vermischung mit der erfindungsgemäßen Färbezubereitung beigemischt wird.

Weiterhin hat es sich als vorteilhaft erweisen, wenn die Färbezubereitungen und/oder die Oxidationsmittelzubereitungen von Oxidationsfärbemitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP) und Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Die erfindungsgemäßen Mittel enthalten weitere Hilfs- und Zusatzstoffe. Vorzugsweise stellen die anwendungsbereiten Mittel fließfähige Zubereitungen dar. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und weiteren nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe.

Bevorzugte anionische Tenside sind Alkylsulfate, Alkylethersulfate, jeweils mit 10 bis 18 C-Atomen in der Alkylgruppe, und polyalkoxylierte Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Ein bevorzugtes zwitterionisches Tensid ist das unter der INCI-Bezeichnung Cocamidopropyl Betaine bekannte Fettsäureamid-Derivat.

In einer weiteren, bevorzugten Ausführungsform der vorliegenden Erfindung enthält das Mittel weiterhin mindestens ein amphoteres Tensid. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet.

Als besonders vorteilhaft haben sich außerdem oberflächenaktive Verbindungen vom Typ nichtionischer Tenside herausgestellt. Solche Verbindungen sind beispielsweise Anlagerungsprodukte von Polyethylenoxid an Fettalkohole, wobei Anlagerungsprodukte von Polyethylenoxid an Fettalkohole besonders bevorzugt sind, die einen durchschnittlichen Ethoxylierungsgrad von 10 bis 45, insbesondere von 12 bis 30 aufweisen, wie beispielsweise Steareth-20, Coceth-15, Oleth-20 oder auch Ceteareth-30, sowie Alkylpolyglykoside entsprechend der allgemeinen Formel RO-(Z)ₓ, wobei R für Alkyl, Z für Zucker sowie x für die Anzahl der Zuckereinheiten steht. Erfindungsgemäß bevorzugte Mittel enthalten als nichtionisches Tensid das Anlagerungsprodukt von Cocos-Fettalkohol an (Poly-)glucose, welches unter der INCI-Bezeichnung Coco-Glucoside bekannt ist.

Die anionischen, zwitterionischen, amphoteren und weiteren nichtionischen Tenside werden in bevorzugt in einer Gesamtmenge von 0,1 bis 45 Gew.%, bevorzugt 1 bis 30 Gew.% und ganz besonders bevorzugt von 1 bis 20 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Es hat sich erfindungsgemäß als vorteilhaft erwiesen, wenn Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Bevorzugt handelt es sich bei dem Verdickungsmittel um ein anionisches, synthetisches Polymer. Bevorzugte anionische Polymere sind Acrylsäure- und/oder Methacrylsäure-Polymere oder - Copolymerisate, die in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten sind. Weiterhin bevorzugt handelt es sich bei dem Verdickungsmittel um ein kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen. Solche Polymere können auch als Copolymere mit nichtionogenen Monomereinheiten, bevorzugt Acrylamid, Methacrylamid, Acrylsäure-C₁-C₄-Alkylester und Methacrylsäure-C₁-C₄-Alkylester, eingesetzt werden. Nichtionische, vollsynthetische Polymere, wie beispielsweise Polyvinylalkohol oder Polyvinylpyrrolidinon, sind ebenfalls als erfindungsgemäße Verdickungsmittel einsetzbar. Weiterhin bevorzugt werden natürlich vorkommende, gegebenenfalls modifizierte Verdickungsmittel eingesetzt. Dazu zählen z.B. Guargums, Skleroglucangums oder Xanthangums, pflanzliche Gums, wie Gummi arabicum, Ghatti-Gummi, Karaya-Gummi, Tragant-Gummi, Carrageen-Gummi, Agar-Agar, Johannisbrotkernmehl, Pektine, Alginate, Stärke, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen. Als anorganische Verdickungsmittel haben sich Schichtsilikate (polymere, kristalline Natriumdisilicate) als besonders geeignet im Sinne der Erfindung erwiesen. Insbesondere Tone, vorzugsweise Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate sind bevorzugt.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, Farbstoffe zum Anfärben des Mittels, Antischuppenwirkstoffe, Wirkstoffe wie Aminosäuren, Oligopeptide und Proteinhydrolysate, Polyphenole und (Pseudo)Ceramide, Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und -distearat sowie PEG-3-distearat, Pigmente, Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Erfindungsgemäß bevorzugte Mittel zur oxidativen Farbveränderung der Haare sind **dadurch gekennzeichnet, dass** sie einen möglichst neutralen pH-Wert besitzen. Eine weitere bevorzugte Ausführungsform der vorliegenden Erfindung besteht darin, dass das anwendungsbereite Mittel einen pH-Wert zwischen 6,0 und 12,0, bevorzugt zwischen 7,0 und 11,5, insbesondere bevorzugt zwischen 8,0 und 11,0 besitzt. Bei den pH-Werten im Sinne der vorliegenden Erfindung handelt es sich um pH-Werte, die bei einer Temperatur von 22°C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß bevorzugte Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol, Triethanolamin, Ammoniak, 1-Aminopropan-2-ol und 2-Amino-2-methylpropan-1-ol.

Die Konfektionierung der erfindungsgemäßen Farbveränderungsmittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der erfindungsgemäßen Komponenten separat verpackt wird; es kann aber bevorzugt sein, die Zubereitung, die zusätzlich mindestens einen Fettsäureester einer ungesättigten Fettsäure mit einem Fettalkohol und mindestens ein Fettsäuretriglycerid enthält, bis zur Anwendung separat zu verpacken. Erfindungsgemäß bevorzugte Mittel sind **dadurch gekennzeichnet, dass** das Mittel unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden, und wobei ein Container ein Färbemittel enthält, welches in einem kosmetischen Träger mindestens ein Oxidationsfarbstoffvorprodukt und zusätzlich mindestens einen Fettsäureester einer ungesättigten Fettsäure mit einem Fettalkohol und mindestens ein Fettsäuretriglycerid enthält, und ein weiterer Container eine Oxidationsmittelzubereitung, enthaltend mindestens ein Oxidationsmittel, enthält. Das Färbemittel besitzt dabei bevorzugt einen pH-Wert zwischen 5,0 und 12,0, bevorzugt zwischen 6,0 und 11,0.

Ein weiterer Gegenstand der vorliegenden Erfindung ist ein Verfahren zur Farb- und/oder Formveränderung keratinischer Fasern, bei dem ein erfindungsgemäßes Mittel gemäß obiger Vorgaben auf das Haar aufgetragen wird, für eine Einwirkzeit von 2 bis 45 min, bevorzugt von 15 bis 30 min auf dem Haar belassen wird, und anschließend das Haar ausgespült wird.

Eine bevorzugte Ausführungsform des erfindungsgemäßen Verfahrens zur Farbveränderung keratinischer Fasern liegt vor, wenn eine Zusammensetzung in einem kosmetischen Träger, enthaltend mindestens ein Oxidationsfarbstoffvorprodukt und zusätzlich mindestens einen Fettsäureester einer ungesättigten Fettsäure mit einem Fettalkohol und mindestens ein Fettsäuretriglycerid, mit einer Oxidationsmittelzubereitung, enthaltend Wasserstoffperoxid, zu einer homogenen Zusammensetzung vermischt, diese auf das Haar aufgebracht wird, für eine Einwirkzeit von 2 bis 120 min, bevorzugt von 3 bis 45 min auf dem Haar belassen wird, und anschließend das Haar ausgespült wird. Die Anwendungstemperaturen bei der erfindungsgemäßen Farbveränderung keratinischer Fasern können in einem Bereich zwischen 15 und 45°C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Bezüglich weiterer bevorzugter Ausführungsformen der erfindungsgemäßen Verfahren gilt mutatis mutandis das zu den erfindungsgemäßen Mitteln Gesagte.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Das anwendungsbereite Mittel wird bei solchen Systemen vom Anwender oder Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein weiterer Gegenstand der vorliegenden Erfindung ist daher eine Mehrkomponentenverpackungseinheit (Kit-of-Parts), enthaltend mindestens zwei getrennt voneinander konfektionierte Container, wobei ein Container (I) eine farbverändernde Zubereitung in einem kosmetischen Träger, enthaltend mindestens ein Oxidationsfarbstoffvorprodukt sowie mindestens einen Fettsäureester einer ungesättigten Fettsäure mit einem Fettalkohol und mindestens ein Fettsäuretriglycerid, enthält und ein zweiter Container (II) eine Oxidationsmittelzusammensetzung, enthaltend mindestens ein chemisches Oxidationsmittel, insbesondere Wasserstoffperoxid, enthält.

Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung der Mittel des ersten Erfindungsgegenstandes zur Verbesserung der Haaroberfläche und zur Verringerung der Schädigung der Haarstruktur bei der oxidativen Farbveränderung menschlicher Haare.

Bezüglich weiterer bevorzugter Ausführungsformen der erfindungsgemäßen Verwendungen gilt mutatis mutandis das zu den erfindungsgemäßen Mitteln Gesagte.

Die nachfolgenden Beispiele erläutern die vorliegende Erfindung, ohne sie auf diesen Umfang einzuschränken.

### Beispiele

### 1) Färbecremes

| Rohstoff | V1 | E1 | E2 |
|---|---|---|---|
| Lanette D [1] | 5,50 | 5,50 | 5,50 |
| Lorol techn. [2] | 2,00 | 2,00 | 2,00 |
| Eutanol G [3] | -- | -- | 1,00 |
| Jojobaöl, goldgelb DAC [4] | -- | 1,00 | 1,00 |
| Mangobutter, ultra refined [5] | -- | 1,00 | 1,00 |
| Eumulgin B 2 [6] | 0,50 | 0,50 | 0,50 |
| Eumulgin B 1 [7] | 0,50 | 0,50 | 0,50 |
| Lamesoft PO 65 [8] | 2,00 | 2,00 | 2,00 |
| Akypo Soft 45HP [9] | 10,00 | 10,00 | 10,00 |
| Texapon K 14 S Special, 70 % [10] | 2,80 | 2,80 | 2,80 |
| Produkt W 37194 [11] | 3,75 | 3,75 | 3,75 |
| p-Toluylendiaminsulfat | 1,16 | 1,16 | 1,16 |
| Resorcin | 0,30 | 0,30 | 0,30 |
| 2-Methylresorcin | 0,13 | 0,13 | 0,13 |
| 3-Aminophenol | 0,06 | 0,06 | 0,06 |
| 2-Amino-3-hydroxypyridin | 0,08 | 0,08 | 0,08 |
| Natriumsilikat 40/42 | 0,50 | 0,50 | 0,50 |
| Ammoniumsulfat, techn. rein | 0,50 | 0,50 | 0,50 |
| Natriumsulfit, wasserfrei, 96% | 0,40 | 0,40 | 0,40 |
| HEDP, 60% | 0,20 | 0,20 | 0,20 |
| Kaliumhydroxid, 50% | 0,90 | 0,90 | 0,90 |
| Ascorbinsäure | 0,05 | 0,05 | 0,05 |
| Natriumsilikat 40/42 | 0,50 | 0,50 | 0,50 |
| Ammoniak, 25 % | 7,50 | 7,50 | 7,50 |
| Parfum | qs | qs | qs |
| Wasser, entsalzt | add 100 | | |

| | | | |
|---|---|---|---|
| [1] C₁₆-C₁₈-Fettalkohol (INCI-Bezeichnung: Cetearyl alcohol) (Cognis) [2] C₁₂-C₁₈-Fettalkohol (INCI-Bezeichnung: Coconut alcohol) (Cognis) [3] 2-Octyldodecanol (INCI-Bezeichnung: Octyldodecanol) (Cognis) [4] (INCI-Bezeichnung: Simmondsia Chinesis Seed Oil) (Gustav Heess) [5] (INCI-Bezeichnung: Mangifera Indica Seed Butter) (Biochemica) [6] C₁₆-C₁₈-Fettalkohol, ethoxyliert (20 EO) (INCI-Bezeichnung: Ceteareth-20) (Cognis) [7] C₁₆-C₁₈-Fettalkohol, ethoxyliert (12 EO) (INCI-Bezeichnung: Ceteareth-12) (Cognis) [8] C₁₂-C₁₈-Alkylpolyglucosid, Glycerylmonooleat (ca. 66 %, INCI-Bezeichnung: Coco-Glucoside, Glyceryl Oleate, Aqua) (Cognis) [10] C₁₂-C₁₄-Alkylether, ethoxyliert (6 EO) Carbonsäure, Natriumsalz (ca. 21 %, INCI-Bezeichnung: Sodium Laureth-6 Carboxylate, Aqua) (KAO) [11] C₁₂-C₁₄-Alkylethersulfat, ethoxyliert (3 EO), Natriumsalz (ca. 70 %, INCI-Bezeichnung: Sodium Myreth Sulfate, Aqua) (Cognis) | | | |

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

### 2) Entwicklerzubereitung EW

| Rohstoff | Gew.-% |
|---|---|
| Dipicolinsäure | 0,10 |
| Dinatriumpyrophosphat | 0,03 |
| HEDP, 60% | 1,50 |
| Texapon NSO [12] | 2,00 |
| Dow Corning DB 110 A [13] | 0,07 |
| Aculyn 33A [14] | 15,00 |
| Ammoniak, 25 % | 0,65 |
| Wasserstoffperoxid, 50 % | 12,00 |
| Wasser, vollentsalzt | ad 100 |

| | |
|---|---|
| [12] Laurylalkohol-diglykolethersulfat, Na-Salz (ca. 28%, INCI-Bezeichnung: Sodium Laureth Sulfate) (Cognis) [13] Acrylpolymer (ca. 28% in Wasser; INCI-Bezeichnung: Acrylates Copolymer) (Rohm & Haas) [14] nicht-ionische Silikonemulsion (INCI-Bezeichnung: Dimethicon) (Dow Corning) | |

### 3) Ausfärbungen:

Die Färbecremes V1 und E1 bis E2 wurden vor der Anwendung mit der Entwicklerlösung EW im Gewichtsverhältnis von 1:1 versetzt und innig vermischt. Pro Gramm Haar (europäisches Humanhaar, Alkinco 6634, #10/2003, A9) wurden 4 g des frisch hergestellten, anwendungsbereiten Färbemittels aufgetragen. Die Einwirkzeit betrug 30 min bei 35°C für die Färbemittel. Danach wurden die Strähnen 30 s lang mit warmem Wasser ausgespült und luftgetrocknet. Die kastanienbraun gefärbten Strähnen zeichneten sich durch glänzende Farben und einen angenehmen Griff aus.

### 4) Trockenkämmarbeit:

Die ausgefärbten Strähnen wurden anschließend hinsichtlich ihrer Trockenkämmarbeit im verhältnis zu unbehandeltem Haar untersucht.

Haarsträhnen (europäisches Humanhaar, Alkinco 6634, #10/2003, A9) wurden mit 4% Natrium Laurethsulfat gewaschen und 3x manuell vorgekämmt. Vor der Anwendung der Testformulierungen wurden die Strähnen 10x automatisch gekämmt und die aufzuwendende Arbeit mit dem force transfer type U1A (Hottinger Baldwin, DE) bestimmt. Nach Anwendung der Mischungen V1/EW, E1/EW bzw. E2/EW wurden die Messungen wiederholt. Es wurden jeweils 20 Haarsträhnen behandelt und gemessen. Als Wert für die Kämmarbeit wurde jeweils das arithmetische Mittel gebildet.

Folgende Ergebnisse wurden erhalten:

| Haarbehandlung | Kämmarbeit |
|---|---|
| unbehandelt | -- |
| gefärbt mit V1 + EW (nicht erfindungsgemäß) - | 46 % |
| gefärbt mit E1 + EW (erfindungsgemäß) | - 33 % |
| gefärbt mit E2 + EW (erfindungsgemäß) | - 12 % |

Die mit den erfindungsgemäßen Mitteln gefärbten Haarsträhnen zeigten eine deutlich verbesserte Kämmarbeit gegenüber den mit Vergleichsmitteln gefärbten Strähnen. Somit schädigen erfindungsgemäße Mittel die keratinische Faser signifikant geringer als die entsprechenden Vergleichsmittel.

## Patentansprüche

1. Mittel zur oxidativen Farbveränderung von keratinischen Fasern, insbesondere menschlichen Haaren, enthaltend in einem kosmetischen Träger mindestens ein Oxidationsfarbstoffvorprodukt, **dadurch gekennzeichnet, dass** das Mittel zusätzlich
(c) mindestens einen Fettsäureester einer ungesättigten Fettsäure mit einem Fettalkohol
und
(d) mindestens ein Fettsäuretriglycerid enthält.

2. Mittel nach Anspruch 1, **dadurch gekennzeichnet, dass** sich der ungesättigte Fettsäurenanteil von Ester (a) von ungesättigten C₂₀ bis C₂₄-Fettsäuren, insbesondere von Gadoleinsäure und/oder Erucasäure, ableitet.

3. Mittel nach einem der Ansprüche 1 bis 2, **dadurch gekennzeichnet, dass** der Ester (a) ein Ester einer ungesättigten Fettsäure mit einem ungesättigten Fettalkohol ist.

4. Mittel nach einem der Ansprüche 1 bis 3, **dadurch gekennzeichnet, dass** sich der Fettalkoholanteil von Ester (a) von ungesättigten, linearen C₁₈ bis C₂₄-Fettalkoholen, insbesondere von Gadoleylalkohol und/oder Erucylalkohol, ableitet.

5. Mittel nach einem der Ansprüche 1 bis 4, **dadurch gekennzeichnet, dass** das Mittel als Fettsäureester (a) das Öl von Samen von Simmondsia chinensis enthält.

6. Mittel nach einem der Ansprüche 1 bis 5, **dadurch gekennzeichnet, dass** das Mittel den/die Fettsäureester (a) in einem Gewichtsanteil von 0,01 bis 15,0 Gew.-%, bevorzugt von 0,05 bis 10,0 Gew.-% und insbesondere von 0,1 bis 5,0 Gew.-%, jeweils bezogen auf das Gesamtgewicht des anwendungsbereiten Mittels, enthält.

7. Mittel nach einem der Ansprüche 1 bis 6, **dadurch gekennzeichnet, dass** das Mittel als Fettsäuretriglycerid (b) ein gemischtes Glycerid von gesättigten und ungesättigten, C₁₆ bis C₂₀-Fettsäuren enthält.

8. Mittel nach einem der Ansprüche 1 bis 7, **dadurch gekennzeichnet, dass** als Fettsäuretriglycerid (b) ein Triglycerid aus den Samen von Mangifera Indica enthalten ist.

9. Mittel nach einem der Ansprüche 1 bis 8, **dadurch gekennzeichnet, dass** das Mittel das/die Fettsäuretriglycerid(e) (b) in einem Gewichtsanteil von 0,01 bis 15,0 Gew.-%, bevorzugt von 0,05 bis 10,0 Gew.-% und insbesondere von 0,1 bis 5,0 Gew.-%, jeweils bezogen auf das Gesamtgewicht des anwendungsbereiten Mittels, enthält.

10. Mittel nach einem der Ansprüche 1 bis 9, **dadurch gekennzeichnet, dass** das Mittel zusätzlich einen verzweigten C₈-C₂₄-Fettalkohol enthält.

11. Mittel nach Anspruch 10, **dadurch gekennzeichnet, dass** der verzweigte Fettalkohol in einem Gewichtsanteil von 0,01 bis 15,0 Gew.-%, bevorzugt von 0,05 bis 10,0 Gew.-% und insbesondere von 0,1 bis 5,0 Gew.-%, jeweils bezogen auf das Gesamtgewicht des anwendungsbereiten Mittels, enthalten ist.

12. Mittel nach einem der Ansprüche 1 bis 11, **dadurch gekennzeichnet, dass** das Mittel zusätzlich ein Oxidationsmittel, ausgewählt aus Wasserstoffperoxid sowie seinen festen Anlagerungsprodukten an organische und anorganische Verbindungen, enthält.

13. Verwendung eines Mittels gemäß einem der Ansprüche 1 bis 12 zur Verbesserung der Haaroberfläche und zur Verringerung der Schädigung der Haarstruktur bei der oxidativen Farbveränderung menschlicher Haare.

14. Kit-of-Parts, enthaltend mindestens zwei getrennt konfektionierte Container, wobei ein Container (I) eine farbverändernde Zubereitung gemäß einem der Ansprüche 1 bis 11 enthält, und ein weiterer Container (II) eine Oxidationsmittelzusammensetzung, enthaltend mindestens ein chemisches Oxidationsmittel, insbesondere Wasserstoffperoxid, enthält.
